# EUROPEAN PATENT APPLICATION

(11) **EP 2 201 939 A1**
(43) Date of publication of application: **30.06.2010**
(21) Application number: 08172946.9
(22) Date of filing: 25.12.2008
(51) Int. Cl.: A61K 9/50, A61K 31/70, A61K 9/00

(54) **Method for producing coated spiramycin and novel formulations containing the same**

(71) Applicant: Zentiva Kimyasal Ürünler Sanayi ve Ticaret A.S., Levent Istanbul 34394 (TR)
(72) Inventor: Karliga, Bekir, 59500 Tekirdag (TR); Turkut, Engin, 59500 Tekirdag (TR); Adiyaman, Mustafa, 59500 Tekirdag (TR)
(74) Representative: Sevinç, Erkan

(57) **Abstract**

A process for the preparation of dispersible particles of spiramycin for use in liquid suspensions for oral uptake is provided. The process comprises coating particles of spiramycin or its pharmaceutically acceptable salts with Eudragit in a direct coating process without prior wet granulation of the active substance. The resulting process thereby provides the novel particles of spiramycin which are very suitable to be used in liquid suspensions prior to oral uptake. The invention further discloses novel formulations containing the powder product obtained from such process wherein bitter taste of spiramycin is completely eliminated, and agglomeration and nonuniform dispersion in oral uptake suspensions are prevented.

## Description

### Field of the Invention

The present invention relates to a method for producing coated particles of spiramycin or its pharmaceutically acceptable salts in order to mask the bitter taste of the same when taken orally. The invention is further directed to obtain dispersible coated spiramycin particles and formulations containing the same whose bitter taste is masked by virtue of a coating process wherein granulation with or without solvents is eliminated, and finer particles which are more convenient for oral uptake and dispersion are provided.

### Background of the Invention

Spiramycin has long been commercially available as a macrolide antibiotic having broad range of activity. This active material is conventionally administered to the patients either by subcutaneous or intradermal routes via injection, or by oral routes which cause difficulties in the ingestion and swallowing of the therapeutic formulation because of its bitterness. This is particularly problematic for the children where they should be administered an oral suspension form of the drug, which is prepared by dispersing an amount of solid spiramycin dosage in a liquid prior to ingestion. It is known that administration by oral routes sometimes even leads to vomiting effect in the children because of excessive bitter taste of spiramycin.

Several attempts have been carried out to mask or at least weaken the bitterness of spiramycin. One of the state of the art techniques, basically consisting of using a suitable sweetener along with a large quantity of sucrose like material, has been disclosed in the French Publication No. 2 696 346. A similar solution is provided in the US Patent Publication No. 5,696,095, wherein bitter taste of the spiramycin is disadvantageously suppressed by large amounts of sweeteners and additional agents (i.e. Potassium acesulfame) along with wet granulation of the formulation components prior to dry state mixing of the same.

Another way of eliminating unpleasant tasting of spiramycin is coating of the formulation granules with more or less complicated coating steps, generally with a suitable and stable polymer or copolymer, as disclosed in the US Patent Application No. 2007/0231397 A1. This document, although is not posing solution for the unpleasant taste of the active ingredients, discloses a method for coating drugs so that to obtain a stable release profile. The method comprises the steps of coating active ingredient containing cores or pellet cores with vinyl (co)polymers in a fluidized bed coating apparatus or a drum coating apparatus and optionally drying and conditioning of the coated pharmaceutical forms in the same apparatus. It is noticed by the inventors, however, that the pharmaceutical forms to be coated are prepared by a wet granulation in the presence of an organic solvent or water which renders the resulting product unsuitable for oral suspension forms because of relatively large granule sizes. Apparently, particle diameters of the active material are crucial for obtaining a uniformly dispersed liquid for oral administration, which otherwise causes nonuniform -agglomerate forming- dispersion in a liquid (e.g. water), and rupture of the granules in the mouth releasing its bitter unpleasant taste.

A method dedicated to provide macrolides suitable for oral administration in a suspension form is disclosed for instance in the US Patent Application No. 2002/0061333 A1. The method for preparing dispersible tablets comprises the steps of: mixing of the active ingredients with a quantity of disintegrants, wet granulation of the resulting mixture in the presence of a wetting liquid containing water and surfactant, drying of the granules thus obtained, dry addition of other excipients and compression of the resulting mixture, wherein macrolide is not coated during the procedure. Forming of a uniformly dispersed drug profile using tablets thus obtained is even harder than those of the previous state of the art products, and the process having a wet granulation step bears the well known drawbacks of the previous references.

US Patent No. 5,403,594 discloses a method for preparing formulations of spiramycin granules by preparing a mixture of an albumin solution and a spiramycin suspension with heating to coagulate albumin and evaporating the solvents to obtain spiramycin granules. Such granules are prepared in accordance with the principles of wet granulation as well, which involves use of solvent(s) and other excipients.

### Summary of the Invention

Although numerous attempts for hiding unpleasant taste of spiramycin have been addressed in the previously known references, there still remains need for a solid spiramiycin form suitable for use in a suspension formulation, which suspension is prepared by simply dispersing a solid dosage form in a liquid prior to administration. This object is achieved by a product and process disclosed in claims 1 and 7 respectively. The invention allows obtaining of coated spiramycin powders which have a particle size distribution in the range of 10 to 600 µm, and most preferably 180 to 350 µm. These ranges of particle sizes are of critical importance for eliminating the particle rupture in the mouth and has not been achieved in the prior art coating procedures. The process of the invention provides these and other advantages by virtue of the process steps including treatment of spiramycin particles with a coating procedure in a fluid bed dryer without a prior wet granulation step. Best results have been achieved through use of conventionally known Eudragit® based polymeric materials.

Accordingly, independent claims attached hereto provide solution to at least the following technical problems of the prior art:
1. Problems associated with preparation of the active substance by wet granulation, which increases consumption of solvent and/or other auxiliary agents, and leads to relatively larger particles (even agglomerates and pellets). Larger entities are known to have tendency to rupture in the mouth leading to release of their bitterness, and are also known as forming non-uniform dispersion in a liquid when used in an oral suspension liquid.
2. Problems relating to the use of sweeteners or other agents in the preparation of the formulations containing active material. Spiramycin, when used as a macrolide in a solid oral formulation, requires large amount of sweeteners for suppressing its excessive bitter taste. This is expensive and particularly disadvantageous with respect to the diabetic patients.
3. Problems associated with the coating material, when active substance is coated so that to mask the bad taste. The coating materials which are not suitable for the oral suspension forms may readily dissolve in the liquid leaving a cloudy dispersion, form non-uniformly dispersed particles and agglomerates, and remain insufficient for masking the bitter taste.

### Objects of the Invention

Objects of the present invention are mainly directed to solve the above identified problems of the prior art, wherein;

One of the objects of the present invention is to provide spiramycin powders coated with a material which does not decompose in an aqueous medium such that leading to a failure in masking the bitter taste of spiramycin,

Another object of the present invention is to provide coated spiramycin powders having substantially smaller particle sizes without forming agglomerates in order to prevent rupture of the material in the mouth upon oral administration,

A further object of the present invention is to eliminate the need for use of sweeteners in the oral solid forms of the spiramycin formulations for suppressing the bitter taste,

Still a further object of the present invention is to provide a process for producing coated spiramycin particles wherein wet granulation of the active substance prior to coating/drying is eliminated, and

Yet another object of the present invention is to provide a process for producing coated spiramycin particles (i.e. powders) having substantially smaller diameters, which are uniformly dispersible and stable in an aqueous liquid without causing turbidity in the suspension.

### Detailed Description of the Invention

The present invention has made it possible to obtain coated spiramycin formulations in powder form which are suitable for deriving uniformly dispersed suspensions particularly for oral use. The inventors unexpectedly discovered that spiramycin, in its crude powder form, can be directly coated in a fluidized bed apparatus without a wet granulation step or any other procedure conventionally used in the art. Such direct coating eliminates the risks relating to decomposition of the active ingredient caused by the exhaustive treatment procedures with solvents and other reagents conducted possibly at high temperatures. The process advantageously introduces new coated spiramycin powders having considerably smaller particle sizes which have never been achieved in the state of the art coating techniques. This is particularly advantageous for preventing rupture of the solid identities in the mouth, formation of non-uniformly dispersed particles in a suspension liquid and heavy ingestion of the suspension after oral uptake.

These and further advantages of the present invention such as cost effectiveness provided by elimination of conventionally known process steps will be apparent to those skilled in the art on account of the description hereinbelow.

The inventors, after conducting performance tests of numerous coating materials, have unexpectedly found out that satisfactory results in the direct coating process of the invention are obtained through use of a polymeric coating material having methacrylate based functional groups. Better results were obtained with cationic polymers having dimethylaminoethyl methacrylate as the functional group. Those materials containing polymers and copolymers of methacrylate functional groups are available in the market under the conventional trade name of Eudragit®. Coating with such material is advantageous at least as regards to the following features:
1. high performance taste masking,
2. very fine and regular particle distribution,
3. no agglomerates, and
4. good dissolution profile (i.e. strength to the acidic environment of the mouth).

The inventors obtained best results with EUDRAGIT® E 12.5, EUDRAGIT® E 100, and EUDRAGIT® E PO of the Eudragit copolymer family.

New pharmaceutical forms of spiramycin for oral administration and for use in an aqueous suspension are obtained by a direct coating process in a fluidized bed apparatus according to following principle steps:
a. dissolving Eudragit in an aqueous mixture comprising a lower alcohol so that to prepare a spray solution,
b. loading solid state crude spiramycin into a fluid bed dryer equipped with an atomizer,
c. spraying said solution onto spiramycin particles, and
d. obtaining coated fine particles of spiramycin in powder form.

Eudragit is dissolved in the presence of a lower alcohol for better coating, wherein the alcohol is selected from the group consisting of methanol, ethanol, propanol, isopropyl alcohol and butanol. Isopropyl alcohol is more preferable.

Particle size of the spiramycin powder loaded into the fluidized bed apparatus is crucial for obtaining uniformly coated particulates having desired particle size profile.

Therefore, solid spiramycin particles loaded into the fluid bed apparatus in powder form are arranged to have a particle size ranging from 5 to 500 µm, more preferably from 10 to 200 µm.

Eudragit spray rate is a parameter which may be defined as the volumetric proportion of spray solution to the inlet air volume in the nozzle of the dryer apparatus. This parameter is kept in the range of 20-80% for obtaining better results. Therefore, other process parameters (i.e. inlet air volume, inlet air temperature etc.) in the fluid bed are adjusted so that to obtain this coating performance. The inlet air temperature is ranging from 35°C to 60°C, more preferably from 40°C to 50°C, and most preferably from 45°C to 47°C.

The inventors further noted that use of silicon dioxide (Aerosil®) considerably increases the free flow of the spiramycin fine particles which also increase the coating performance of the process. Aerosil is added into the fluid bed apparatus along with the crude spiramycin powders.

The resulting product coated according to the process of the invention may have a particle size distribution in the range of 10 to 600 µm, which range is noted to be the uppermost limit of particle diameters for eliminating the ingestion-swallowing and rupture problems of the art. Therefore the process of the present invention disclosed in claim 7 gives satisfactory results to obtain aforementioned particle size ranges.

However, the inventors report that a range defined in between 180 µm and 350 µm is the most ideal particle size distribution for especially oral uptake of the children. This range has also advantageously provided better performance in the product for to be uniformly dispersible in an aqueous liquid medium without formation of turbidity and agglomeration. On the other hand, the resulting product in particulate form is completely eliminated of the severe bitter taste of spiramycin even in a liquid suspension prepared prior to administration. This is apparently because of relatively small particle size distribution of the product - eliminating agglomeration and rupture of the particles in the mouth -, and good and stable coating performance of eudragit - eliminating dissolution of the coating material in the suspension and also in the acidic environment of the mouth -.

In a further aspect, there is provided novel formulations containing spiramycin particles of the invention as disclosed in claim 4. Accordingly, the coated particles of the invention may optionally be combined with pharmaceutically acceptable excipients for obtaining formulations to be used in oral suspensions. Such excipients may include but not limited to Aroma (tutti frutti), alveo sugars, colloidal anhydrous silica, titanium dioxide and xanthan gum.
The following examples are given for showing taste masking, suspension turbidity-dispersion performances of alternative coating materials compared to the Eudragit copolymers used in the present invention.

### A. Preparation of the Test Solution

A 2 L flask was poured 16.6 ml of concentrated HCI and filled with distilled water in its entirety. This acidic solution was kept for the further experiments in which the suspensions of coated spiramycin are prepared.

### B. Preparation of the Spray Suspension

0.9 kg of deionized water was poured into a vessel containing 13.5 kg acetone and 20.25 kg IPA. The aqueous solution was stirred with a mechanical agitator for half an hour at ambient temperature. This solvent mixture was then slowly added 4.5 kg of the coating material in a manner such that after each addition the material is waited to be dissolved completely in the solution. After completion of such addition, the suspension thus obtained was stirred for 1 hour at ambient temperature and filtered through a 0.2 mm filter.

### Comparative Example 1

400 g of spiramycin crude powder was loaded into a fluidized bed dryer apparatus together with 20 g of Aerosil. A spray solution prepared according to the procedure given in Part B and comprising 4,500 g of Stearic acid (Malysia Edenor, Cognis) was sprayed onto the crude particles flowing through the fluidized bed wherein inlet air temperature was adjusted to 47°C and coating solution : air mixture proportion was adjusted to 20%. Coated spiramycin product was collected into a vessel.

1 L of the test solution prepared according to the procedure given in Part A was poured into a flask and temperature was adjusted to 37.0 ± 0.5°C. Then 750.000 ID equivalent coated spiramycin was added into the flask and the suspension was stirred for half an hour at 100 rpm.

### Features of the resulting product:

Slightly bitter taste,
Non-uniform dispersion (white particles floated on the suspension surface, agglomerates have been formed),
Cloudy suspension
Particles ruptured in the mouth

### Comparative Example 2

400 g of spiramycin crude powder was loaded into a fluidized bed dryer apparatus together with 20 g of Aerosil. A spray solution prepared according to the procedure given in Part B and comprising 4,500 g of Stearyl alcohol (Lanette 18, Cognis) was sprayed onto the crude particles flowing through the fluidized bed wherein inlet air temperature was adjusted to 47°C and coating solution : air mixture proportion was adjusted to 20%. Coated spiramycin product was collected into a vessel.

1 L of the test solution prepared according to the procedure given in Part A was poured into a flask and temperature was adjusted to 37.0 ± 0.5°C. Then 750.000 IU equivalent coated spiramycin was added into the flask and the suspension was stirred for half an hour at 100 rpm.

### Features of the resulting product:

Slightly bitter taste
Non-uniform dispersion (white particles floated on the suspension surface)
Clear suspension
Particles not ruptured in the mouth

### Comparative Example 3

400 g of spiramycin crude powder was loaded into a fluidized bed dryer apparatus together with 20 g of Aerosil. A spray solution prepared according to the procedure given in Part B and comprising 4,500 g of Glycerol distearate (CUTINA® AGS, Cognis) was sprayed onto the crude particles flowing through the fluidized bed wherein inlet air temperature was adjusted to 47°C and coating solution : air mixture proportion was adjusted to 20%. Coated spiramycin product was collected into a vessel.

1 L of the test solution prepared according to the procedure given in Part A was poured into a flask and temperature was adjusted to 37.0 ± 0.5°C. Then 750.000 IU equivalent coated spiramycin was added into the flask and the suspension was stirred for half an hour at 100 rpm.

### Features of the resulting product:

Very slightly bitter taste,
Non-uniform dispersion (white particles floated on the suspension surface),
Clear suspension
Particles not ruptured in the mouth

### Comparative Example 4

400 g of spiramycin crude powder was loaded into a fluidized bed dryer apparatus together with 20 g of Aerosil. A spray solution prepared according to the procedure given in Part B and comprising 4,500 g of EUDRAGIT ® E 100 was sprayed onto the crude particles flowing through the fluidized bed wherein inlet air temperature was adjusted to 47°C and coating solution : air mixture proportion was adjusted to 20%. Coated spiramycin product was collected into a vessel.

1 L of the test solution prepared according to the procedure given in Part A was poured into a flask and temperature was adjusted to 37.0 ± 0.5°C. Then 750.000 IU equivalent coated spiramycin was added into the flask and the suspension was stirred for half an hour at 100 rpm.

### Features of the resulting product:

No bitter taste,
Relatively uniform dispersion (white particles are regularly dispersed within the suspension liquid, agglomerates have not been formed),
Clear suspension
Particles not ruptured in the mouth

### RESULTS

The spiramycin particles obtained by the direct coating process of the present invention are at least advantageous as regards to the particle sizes thereof suitable for preparing oral suspension forms. Resulting particles with the aforementioned particle sizes do not allow rupture in the mouth during oral uptake and are easy to ingest as may be perceived in the comparative examples.

On the other hand, a further surprising effect has been noticed with the spiramycin particles coated with Eudragit. Although the alternative coating materials have been eliminating the bitter taste in certain extent, best results have been achieved with Eudragit polymers which have completely eliminated the drawbacks well known in the art. The oral suspension obtained by dispersion of the eudragit coated spiramycin powders have been at least improved in terms of the turbidity (indication of solubility of the particles in the suspension liquid) and dispersion performances compared to other suspensions.

## Claims

1. Dispersible particles of spiramycin or pharmaceutically acceptable salts thereof **characterized in that** the particles are coated with a polymeric material of EUDRAGIT® and particles sizes of said particles are being in the range from 180 µm to 350 µm.

2. The particles according to claim 1 wherein the polymeric material is selected from the group consisting of EUDRAGIT® E 12.5, EUDRAGIT® E 100, and EUDRAGIT® E PO.

3. A pharmaceutical formulation comprising dispersible spiramycin particles of claim 1.

4. The formulation according to claim 3 wherein the formulation further comprises a pharmaceutically acceptable excipient selected from the group consisting of aroma (tutti frutti), alveo sugars, colloidal anhydrous silica, titanium dioxide and xanthan gum.

5. Use of the dispersible particles of claim 1 as an antibiotic for treatment of bacterial infections.

6. Dispersible particles of claim 1 for use in a liquid suspension for oral administration.

7. A process for producing dispersible particles of spiramycin or its pharmaceutically acceptable salts for oral uptake, comprising the steps of:
dissolving a polymeric material of EUDRAGIT® in a liquid mixture to obtain a spray solution, the mixture comprising a lower alcohol as a solvent,
loading solid state crude spiramycin powder into a fluid bed dryer apparatus equipped with an atomizer, the spiramycin powder loaded into said apparatus having particle sizes ranging from 5 µm to 500 µm,
spraying the solution onto the spiramycin powders which are in free flow movement through the fluid bed, and
obtaining coated fine particles of spiramycin having particle sizes ranging from 10 µm to 600 µm.

8. The process according to claim 7 wherein the lower alcohol is selected from the group consisting of methanol, ethanol, propanol, isopropyl alcohol and butanol.

9. The process according to claim 7 wherein the spiramycin particles obtained from the process are having particle sizes ranging from 180 µm to 350 µm.

10. The process according to claim 7 wherein the solid state spiramycin powder loaded into the fluid bed is having particle sizes ranging from 10 to 200 µm.

11. The process according to claim 7 wherein the spray solution : inlet air volume proportion in the atomizer is adjusted to value ranging from 20% to 80%.

12. The process according to claim 7 or 11 wherein inlet air temperature into the fluid bed apparatus is adjusted to a value ranging from 35°C to 60°C.

13. The process according to claim 12 wherein inlet air temperature ranges from 45°C to 47°C.

14. The process according to claim 7 wherein crude spiramycin powder is added silicon dioxide to improve free flow within the fluid bed.
